# EUROPEAN PATENT APPLICATION

(11) **EP 4 487 769 A1**
(43) Date of publication of application: **08.01.2025**
(21) Application number: 23183419.3
(22) Date of filing: 04.07.2023
(51) Int. Cl.: A61B 5/11, A61B 5/369, A61B 5/00

(54) **THERAPY FOR THE TREATMENT OF NERVOUS SYSTEM DISORDERS USING CROSS-PATIENT TREMOR DETECTION THROUGH MACHINE LEARNING**

(71) Applicant: BIOTRONIK SE & Co. KG, 12359 Berlin (DE)
(72) Inventor: Schulze-Luckow, Sebastian, 10715 Berlin (DE); Kibler, Andrew B., Lake Oswego, 97035 (US)
(74) Representative: Biotronik Corporate Services SE

(57) **Abstract**

The present invention relates to a computing device (1), comprising: a storage medium (10); and processing circuitry (13) operably coupled to the storage medium (10) and configured to: receive first data sensed by a medical device, and second data sensed by at least one wearable device apply a machine learning model, trained using first and second data for a plurality of patients, to the received first and second data to: determine, based on the machine learning model, that an episode of tremor has occurred in the patient; and determine a level of confidence in the determination that the episode of tremor has occurred in the patient; determine that the level of confidence in the determination that the episode of tremor has occurred in the patient is greater than a predetermined threshold; and in response to determining that the level of confidence is greater than the predetermined threshold, output, for display to a user, at least a portion of the first data, a first indication that the episode of tremor has occurred in the patient, and a second indication of the level of confidence that the episode of tremor has occurred in the patient.

## Description

The present invention relates to computing systems and methods for detecting tremors in patients.

Typical symptoms of diseases like Parkinson's are tremors. A tremor is an involuntary, often rhythmic, muscle contraction and relaxation involving back and forth movements of one or more body parts. It is one of the most common involuntary movements, and frequently occurs in the hands of a patient, but it can also affect other body parts such as arms, eyes, face, head, vocal folds, trunk, and legs.

Known solutions for diagnosing/treating tremors require visiting a physician for clinical presentation, magnetic resonance therapy of the brain after medical diagnosis, and measurement of muscle activity by means of electrodes in the clinic.

However, all of the above measures typically involve visiting a physician. The typical population of patients is regularly older than 65 years, the repeated visit of the doctor is necessary for the diagnosis of this population. Thus, particularly, only a temporary measurement (time window) is provided, not a permanent monitoring, so that an insufficient diagnosis is a possible outcome. Furthermore, the diagnosis depends on the experience of the physician, wherein such diagnosis usually confirms that a tremor is either present or not, and may describe the quality of the tremor as severe or mild - but will regularly not be given as objectively measured value. Therefore, existing solutions are often partially unnecessary, expensive, time consuming, i.e. they tie up personnel, not comparable to the advance of the state of knowledge, and possibly prone to error as the physician evaluates.

Therefore, based on the above, the problem to be solved by the present invention is to provide a computing system and a method to improve detection of involuntary body movements such as tremors.

This problem is solved by a computing system having the features of claim 1, and by a computing system having the features of claim 13 as well as by a method having the features of claim 14. Preferred embodiments of these aspects of the present invention are stated in the corresponding dependent claims and are described below.

According to claim 1 a computing system is disclosed, comprising:
a storage medium; and
processing circuitry operably coupled to the storage medium and configured to:
   receive first data sensed by a medical device, and second data sensed by at least one wearable device
   apply a machine learning model, trained using first and second data for a plurality of patients, to the received first and second data to:
      determine, based on the machine learning model, that an episode of tremor has occurred in the patient; and
      determine a level of confidence in the determination that the episode of tremor has occurred in the patient;
      determine that the level of confidence in the determination that the episode of tremor has occurred in the patient is greater than a predetermined threshold; and in response to
      determining that the level of confidence is greater than the predetermined threshold, output, for display to a user, at least a portion of the first data, a first indication that the episode of tremor has occurred in the patient, and a second indication of the level of confidence that the episode of tremor has occurred in the patient.

According to a preferred embodiment of the computing system, the first data comprises at least one of: data indicative of neural activity, an electroencephalogram (EEG) waveform, an electrocortigram (ECoG) waveform.

Furthermore, according to a preferred embodiment of the computing system, the second data comprises one of: data concerning motion, particularly of a patient wearing the wearable device; a temperature of a patient, particularly wearing the wearable device; blood pressure, particularly of a patient wearing the wearable device.

Particularly said motion can be a motion of one of: a limb of a patient, a head of a patient, a hand of a patient. The data concerning motion can be one or more acceleration time series being indicative of an acceleration of a body part of the patient, e.g., of one of the above-stated body parts such as limb, head, hand etc. of the patient.

Furthermore, according to a preferred embodiment of the computing system, the at least one portion of the first data comprises an electroencephalogram (EEG) waveform, and wherein the first indication that the episode of tremor has occurred in the patient comprises an annotation to the EEG waveform.

Further, according to a preferred embodiment of the computing system, the second indication comprises one or more of: a color, an image, a light, a sound, a textual notification.

In yet another preferred embodiment of the computing system, the processing circuitry is further configured to receive, from the user, a selection of a tremor type, wherein to apply the machine learning model to the received first and second data to determine that an episode of tremor has occurred in the patient, the processing circuitry is configured to apply the machine learning model to the received first and second data to determine that an episode of tremor of the selected tremor type has occurred in the patient, and wherein to output the at least one portion of the first data, the first indicator that the episode of tremor has occurred in the patient, and the second indicator of the level of confidence that the episode of tremor has occurred in the patient, the processing circuitry is configured to output the at least one portion of the first data, a first indicator that the episode of tremor of the selected tremor type has occurred in the patient, and the second indicator of the level of confidence that the episode of tremor of the selected tremor type has occurred in the patient.

For instance, typical tremor types are resting tremor, postural tremor, kinetic tremor, task-specific tremor, and intention tremor. Resting tremor occurs when a body part is at complete rest against gravity. Tremor amplitude decreases with voluntary activity. Examples of resting tremor are / occur as: Drug-induced parkinsonism (neuroleptics, metaclopromide, and phenothiazines), long-standing essential tremor, Parkinson's disease, Parkinson's plus syndromes (rest tremor is less common), or Wilson's disease.

Postural tremor occurs during maintenance of a position against gravity and increases with action. Action or kinetic tremor occurs during voluntary movement. Examples of postural and action tremors are /occur due to: Essential tremor (primarily postural), metabolic disorders (thyrotoxicosis, pheochromocytoma, hypoglycemia), drug-induced parkinsonism (lithium, amiodarone, 0-adrenergic agonists), toxins (alcohol withdrawal, heavy metals), neuropathic tremor (neuropathy). Task-specific tremor emerges during a specific activity. An example of this type is primary writing tremor. Intention (or terminal) tremor manifests as a marked increase in tremor amplitude during a terminal portion of targeted movement. Examples of intention tremor include cerebellar tremor and multiple sclerosis tremor.

Furthermore, according to a preferred embodiment of the computing system, the processing circuitry is configured to determine that the user is a basic user, wherein, to output the at least one portion of the first data, the first indicator that the episode of tremor has occurred in the patient, and the second indicator of the level of confidence that the episode of tremor has occurred in the patient, the processing circuitry is configured to output, in response to determining that the user is a basic user, the first indicator that the episode of tremor has occurred in the patient, the second indicator of the level of confidence that the episode of tremor has occurred in the patient, and one or more of: an electroencephalogram (EEG) waveform of the patient; a first representation of a first EEG waveform presenting an episode of tremor; and a second representation of a second EEG waveform presenting normal behavior.

Furthermore, in a preferred embodiment of the computing system, the processing circuitry is configured to determine that the user is an advanced user, wherein, to output the at least one portion of the first data, the first indicator that the episode of tremor has occurred in the patient, and the second indicator of the level of confidence that the episode of tremor has occurred in the patient, the processing circuitry is configured to output, in response to determining that the user is an advanced user, the first indicator that the episode of tremor has occurred in the patient, the second indicator of the level of confidence that the episode of tremor has occurred in the patient, and one or more of: an electroencephalogram (EEG) waveform of the patient; a start time of the episode of tremor; a stop time of the episode of tremor.

Further, according to a preferred embodiment of the computing system, the machine learning model trained using first and second data for the plurality of patients comprises a machine learning model trained using a plurality of electroencephalogram (EEG) waveforms, each EEG waveform labeled with one or more episodes of tremor in a patient of the plurality of patients.

In a preferred embodiment of the computing system, to apply the machine learning model to the received first and second data, the processing circuitry is configured to apply the machine learning model to at least one of: electroencephalogram (EEG) data of the patient; the characteristics correlated to tremor in the patient; a type of the tremor in the patient; an activity level of the implantable medical device; an input impedance of the implantable medical device; a battery level of the implantable medical device; motion data correlated to tremor in the patient; temperature of the patient correlated to tremor in the patient, blood pressure correlated to tremor in the patient.

Preferably, according to a further embodiment, to output the at least one portion of the first data, the processing circuitry is configured to: identify a subsection of an electroencephalogram (EEG) of the patient, wherein the subsection comprises EEG data for a first time period prior to the episode of tremor, a second time period during the episode of tremor, and a third time period after the episode of tremor, and wherein a length of time of the EEG of the patient is greater than the first, second, and third time periods; and output the subsection of the EEG.

Furthermore, in a preferred embodiment of the computing system, the predetermined threshold is a first predetermined threshold, and wherein the processing circuitry is further configured to determine whether the level of confidence in the determination that the episode of tremor has occurred in the patient is greater than a second predetermined threshold, the second predetermined threshold greater than the first predetermined threshold, wherein in response to determining that the level of confidence is greater than the predetermined threshold, to output, the at least one portion of the first data, the first indication, and the second indication, the processing circuitry is configured to: in response to determining that the level of confidence is greater than the first predetermined threshold but not greater than the second predetermined threshold, output, the at least one portion of the first data, the first indication, and an indication of a medium level of confidence that the episode of tremor has occurred in the patient; in response to determining that the level of confidence is greater than the first predetermined threshold and greater than the second predetermined threshold, output, the at least a portion of the first data, the first indication, and an indication of a high level of confidence that the episode of tremor has occurred in the patient.

According to a further aspect of the present invention, a computing system is disclosed, the computing system comprising:
means for receiving, by a computing system comprising processing circuitry and a storage medium, first data sensed by a medical device and second data sensed by a wearable device;
means for applying, by the computing system, a machine learning model, trained using first and second data for a plurality of patients, to the received first and second data to:
   determine, based on the machine learning model, that an episode of tremor has occurred in the patient; and
   determine a level of confidence in the determination that the episode of tremor has occurred in the patient;
   means for determining that the level of confidence in the determination that the episode of tremor has occurred in the patient is greater than a predetermined threshold; and means for outputting, by the computing system and for display to a user, at least a portion of the first data, a first indication that the episode of tremor has occurred in the patient, and a second indication of the level of confidence that the episode of tremor has occurred in the patient.

According to a preferred embodiment of the computing system, the first data comprises at least one of: data indicative of neural activity, an electroencephalogram (EEG) waveform, an electrocortigram (ECoG) waveform. Furthermore, according to a preferred embodiment of the computing system, the second data comprises one of: data concerning motion, temperature, blood pressure.

According to yet another aspect of the present invention a method (particularly using a computing system according to the present invention) is disclosed, comprising:
Receiving, by a computing system comprising a storage medium and
processing circuitry operably coupled to the storage medium,
first data sensed by a medical device, and second data sensed by at least one wearable device;
applying, by the computing system, a machine learning model, trained using first and second data for a plurality of patients, to the received first and second data to:
   determine, based on the machine learning model, that an episode of tremor has occurred in the patient; and
   determine a level of confidence in the determination that the episode of tremor has occurred in the patient;
   determining that the level of confidence in the determination that the episode of tremor has occurred in the patient is greater than a predetermined threshold; and in response to determining that the level of confidence is greater than the predetermined threshold, outputting, by the computing system and for display to a user, at least a portion of the first data, a first indication that the episode of tremor has occurred in the patient, and a second indication of the level of confidence that the episode of tremor has occurred in the patient.

According to a preferred embodiment of the method according to the present invention, the first data comprises at least one of: data indicative of neural activity, an electroencephalogram (EEG) waveform, an electrocortigram (ECoG) waveform. Furthermore, according to a preferred embodiment of the method, the second data comprises one of: data concerning motion, temperature, blood pressure.

The method according to the present invention preferably uses a computing system according to the present invention. The method can be further characterized by the individual features and individual embodiments of the computing system described herein and as claimed in claims 1 to 12.

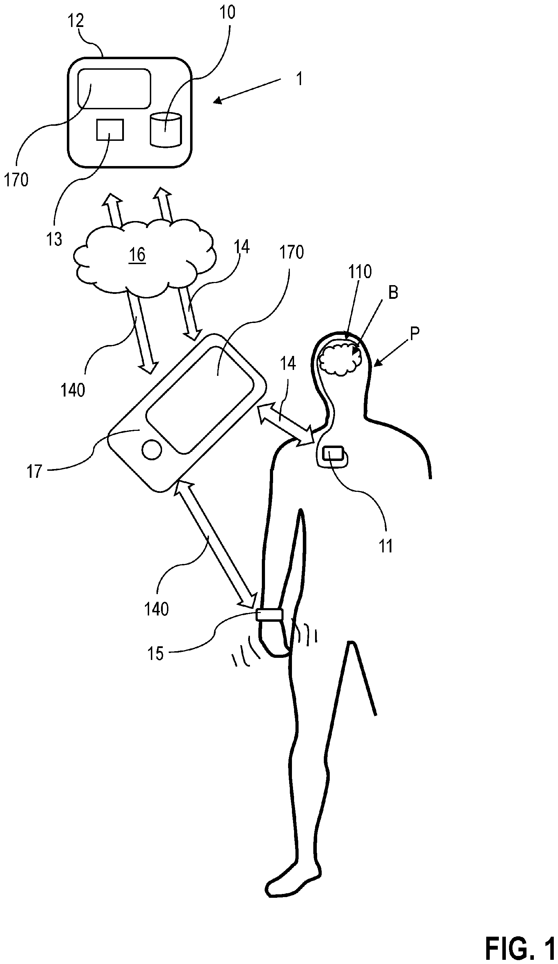

In the following, embodiments of the aspects of the present invention as well as further features and advantages of the present invention are described with reference to the Figures, wherein
- Fig. 1: an embodiment of a computing system and method according to the present invention using machine learning to detect tremors in a patient.

Fig. 1 shows a schematic illustration of an embodiment of a computing system 12 according to the present invention, comprising: a storage medium 10 and processing circuitry 13 operably coupled to the storage medium 10 and configured to: receive first data sensed by a medical device 11, and second data sensed by at least one wearable device 15. The computing system 12 is further configured to apply a machine learning model, trained using first and second data for a plurality of patients, to the received first and second data to: determine, based on the machine learning model, that an episode of tremor has occurred in the patient; and determine a level of confidence in the determination that the episode of tremor has occurred in the patient; determine that the level of confidence in the determination that the episode of tremor has occurred in the patient is greater than a predetermined threshold; and in response to determining that the level of confidence is greater than the predetermined threshold, output, for display to a user, at least a portion of the first data, a first indication that the episode of tremor has occurred in the patient, and a second indication of the level of confidence that the episode of tremor has occurred in the patient.

Particularly, the machine learning model can be based on deep learning and other artificial intelligence (AI) techniques, and performs tremor detection, while also providing measures for visualizing an output of the machine learning model.

As indicated in Fig. 1 medical device 11 is preferably an implantable medical device 11 such as a brain pacemaker and may comprise at least one electrode 110 to sense EEG signals so as to provide EEG waveforms of the brain B of the patient P as part of said first data. Furthermore, the second data can comprise data relating to a motion of the patient P as described herein and/or temperature of the patient P and/or blood pressure of the patient P. Furthermore, computing system 12 is preferably part of a system 1 that also comprises the medical device 11 and the wearable device 15. This system forms a further aspect of the present invention. Particularly, medical device 11 and/or wearable device 15 can be configured to communicate with computing system 12 via an external device 17, particularly via wireless radio connections 14, 140, respectively. External device 17 may be a computing device (e.g. an external patient device), but can also be a programmer, an external monitor, a mobile device, such as a mobile phone, a smart phone, a laptop, a tablet computer, a personal digital assistant (PDA) etc., configured for use in settings such as a home, clinic, or hospital, and may further be configured to communicate with medical device 11 and wearable device 15 via wireless (e.g. radio) communication. For example, external device 17 may be coupled to computing system 12 via a network 16. Computing system 12 may include a remote patient monitoring system. External device 17 can comprise a display 170 for displaying information to user such as the patient P and/or a physician/clinician. According to a preferred embodiment, regarding all embodiments described herein wearable device 15 may generally comprise the functionality of the external device 17 so that external device 17 can be omitted and all its functions are performed by wearable device 15.

According to an alternative embodiment, medical device 11 may also be an external medical device that is not implanted into the patient and may itself sense the first data or receive it from a further source. Such an external medical device may be positioned externally to patient P (e.g., positioned on the skin of patient P) and may carry out any or all of the functions described herein with respect to the implantable medical device 11.

Particularly, a user, such as a physician, technician, surgeon, electro physiologist, or other clinician, may interact with external device 17 to retrieve physiological or diagnostic information from the implantable medical device 11 and/or wearable device 15. Furthermore, a user, such as patient P or a clinician as described above, may also interact with external device 17 to program the implantable medical device 11, e.g., select or adjust values for operational parameters of the implantable medical device 11.

Furthermore, computing system 12 can comprise a handheld computing device, computer workstation, server or other networked computing device, smartphone, tablet, or external programmer that includes a user interface 170 (e.g. comprising a display) for presenting information to and receiving input from a user. Particularly, computing system 12 has implemented therein, a neural network, a deep learning system, or another type of predictive analytics system. A user, such as a physician, technician, surgeon, electro-physiologist, or other clinician, may operate computing system 12 so as to retrieve physiological or diagnostic information from the implantable medical device 11. A user may also interact with computing system 12 to program the implantable medical device 11, e.g., select values for operational parameters of the implantable medical device 11. Such programming can alternatively, or in addition, also be carried out by means of the external device 17.

Network 16 can provide computing system 12 and implantable medical device 11 access to the Internet, and may provide a communication framework that allows the devices 11, 15, 17, 12 to communicate with one another. Particularly, network 16 may be a private network that provides a communication framework that allows computing system 12, implantable medical device 11, wearable device 15, and/or external device 17 to communicate with one another but can isolate one or more of computing system 12, implantable medical device 11, external device 17 from devices external to network 16 for security purposes. Particularly, the communications between computing system 12, implantable medical device 11, and external device 17 are encrypted. Furthermore, external device 17 and computing system 12 can communicate via wireless communication over network 16 using any techniques known in the art. Particularly, computing system 12 is a remote system that is configured to communicate with external device 17 via an intermediary device located in network 16, such as a local access point, wireless router, or gateway. However, external device 17 and computing system 12 may also communicate with one another directly, e.g. based on wireless radio communication using e.g. Bluetooth or BLE protocols. Other communication techniques are also conceivable. Furthermore, according to yet another preferred embodiment, computing system 12 can be configured to communicate with the medical device 11 and/or the wearable device 15 without the use of external device 17 which can be omitted in this case. In such an embodiment, computing system 12 can be a mobile device and may be one of a mobile phone, a smart phone, a laptop, a tablet computer, a personal digital assistant (PDA), an external patient device, a programmer, an external monitor etc.

Particularly, external device 12 may be used to program commands or operating parameters into implantable medical device 11 for controlling its functioning (e.g., when being configured as a programmer for the implantable medical device 11). Particularly, external device 17 can be configured to retrieve first data from implantable medical device 11 and second data from wearable device 15. Such retrieving of data can occur automatically according to a schedule and/or may occur in response to a remote or local user command. wireless communication used by implantable medical device 11, wearable device 15 and external device 17 to this end can include radio communication, e.g. based on Bluetooth, WiFi, or medical implant communication service (MICS). Furthermore, external device 17 may include a user interface configured to allow patient P, a clinician, or another user to remotely interact with implantable medical device 11.

The computing system 12 / system 1 is configured to provide information and particularly visualize an output of its implemented machine learning model for detecting tremor in a patient P. Particularly, computing system 12 / system 1 functions to simplify the conclusions drawn by the machine learning model with respect to the detection of tremor in patient P. Further, computing system 12 / system 1 is configured to provide such information (e.g. via displays 170 of computing system 12 or external device 17 in a manner adapted to users of differing ability, including subject matter experts and non-experts alike.

As indicated above, computing system 12 further determines a level of confidence in the determination that an episode of tremor has occurred in patient P. In response to determining that the level of confidence is greater than a predetermined threshold, computing system 12 particularly displays, to a user, a portion of the first data, particularly an EEG waveform, an indication that the episode of tremor has occurred, and an indication of the level of confidence that the episode of tremor has occurred. Particularly, computing system 12 can provide more detailed information to advanced users and less detailed information to basic users. Furthermore, particularly, computing system 12 receives first data including EEG data sensed by implanted medical device 11 and second data provided by wearable device 15, and a selection of a tremor type from a user. Computing system 12 applies the machine learning model to the received first and second data to determine, based on the machine learning model of computing system 12, that an episode of tremor of the selected type has occurred in patient P and a level of confidence in the determination that the episode of tremor of the selected type has occurred. Particularly, computing system 12 can output, for display, at least a portion of the first data comprising at least EEG data, a first indication that the episode of tremor of the selected type has occurred in patient P, and a second indication of the level of confidence that the episode of tremor of the selected type has occurred in patient P.

The present invention provides specific improvements to the field of machine learning systems that perform tremor detection and classification due to providing information and visualization of the analysis performed by the employed machine learning algorithm Accordingly, the invention supports more accurate and faster diagnosis and classification of tremor in patients, while reducing the amount of expertise required by clinicians to diagnose and provide therapy for tremor / Parkinson's disease.

Furthermore, particularly, processing circuitry 13 can include fixed function circuitry and/or programmable processing circuitry. Processing circuitry 13 may include any one or more of: a microprocessor, a controller, a digital signal processor (DSP), an application specific integrated circuit (ASIC), a field-programmable gate array (FPGA), discrete or analog logic circuitry. Particularly, processing circuitry 13 can include multiple components, such as any combination of one or more microprocessors, one or more controllers, one or more DSPs, one or more ASICs, or one or more FPGAs, as well as other discrete or integrated logic circuitry. The functions provided by processing circuitry 13 as described herein can be embodied as software, firmware, hardware or any combination thereof.

Particularly, implantable medical device 11 can transmit one or more segments of the EEG data / waveform and particularly of the second data in response to detecting, e.g. via feature delineation, an episode of tremor. Furthermore, implantable medical device 11 can transmit one or more segments of the EEG data / waveform in response to instructions from external device 17 (e.g., when patient P experiences tremor and inputs a command to external device 17 instructing internal medical device 11 to upload the first data (e.g. EEG data and particularly other data described herein) and second data for analysis by a monitoring center or clinician). The first and second data (particularly EEG data) may be processed by computing system 12 based on the machine learning model to detect and classify tremor as described in detail below.

Furthermore, particularly, the machine learning model implemented by computing system 12 is trained with training data that comprises first and second data, particularly EEG data, for a plurality of patients labeled with descriptive metadata. For example, during a training phase, computing system 12, particularly processing circuitry 13, processes a plurality of first data (e.g. EEG waveforms) and second data. Typically, the plurality of first and second data (particularly EEG waveforms) are from a plurality of different patients. Each EEG waveform and each second data (e.g. motion, particularly acceleration data, temperature or blood pressure) is labeled with one or more episodes of tremor of one or more types. For example, a training EEG waveform may include a plurality of segments, each segment labeled with a descriptor that specifies an absence of tremor or a presence of a tremor of a particular classification. In some examples, a clinician labels the presence of tremor in each first data item, particularly EEG waveform, and/or second data item by hand. In some examples, the presence of tremor in each first data / EEG waveform and second data is labeled according to classification by a feature delineation algorithm. Computing system 12 with machine learning model implemented therein can operate to convert the training data into vectors and tensors (e.g., multi-dimensional arrays) upon which computing system 12 can apply mathematical operations, such as linear algebraic, nonlinear, or alternative computation operations.

Particularly, computing system 1 uses the training data to teach the machine learning model to weigh different features depicted in the first data, particularly EEG waveform(s), and second data. Particularly, computing system 12 uses the EEG data to teach the machine learning model to apply different coefficients that represent one or more features in an EEG as having more or less importance with respect to an occurrence of a tremor of a particular classification. Particularly, the same applies to other first data and the second data. By processing numerous such EEG waveforms (and particularly other first or second data) labeled with episodes of tremor, computing system 12 and its processing circuitry 13 can train a machine learning model to receive first data, particularly EEG data, and second data from a patient P that has not been analyzed previously, and process such data to detect the presence or absence of tremor of different classifications in the patient with a high degree of accuracy. Typically, the greater the amount of first and second data on which the machine learning model implemented by the computing system 12 is trained, the higher the accuracy of the machine learning model in detecting or classifying tremor in new first and second data, particularly in EEG data.

After computing system 12 has trained the machine learning model, computing system 12 can receive patient data, such as EEG data, for a particular patient P. Computing system 12 then applies the trained machine learning model to the actual first and second data to detect an occurrence of an episode of tremor in patient P. Further, computing system 12 applies the trained machine learning model to the patient data to classify the episode of tremor in patient as indicative of a particular type of tremor. Particularly, the computing system 12 can output a determination that the episode of tremor is indicative of a particular type of tremor, as well as a level of confidence in the determination. In response to determining that the level of confidence in the determination is greater than a predetermined threshold (e.g., 50%, 75%, 90%, 95%, 99%), computing device 12 may classify that the episode of tremor as the particular type of tremor and output, for display to a user, at least a portion of the first data (e.g., a portion of an EEG) during which the episode of tremor occurred, and particularly also second data, as well as a first indication that the episode of tremor has occurred in patient P, and a second indication of the level of confidence in the determination that the episode of tremor has occurred.

Furthermore, particularly, computer system 12 can process one or more features of EEG data or of other first and second data instead of the raw EEG or first and second data itself. The one or more features can be obtained via feature delineation e.g. performed by implantable medical device 11 and particularly wearable device 15 depending on the data being first or second data. Said features may include, e.g., one or more amplitudes of one or more features of an EEG of the patient, or an interval between features of the EEG of the patient. Particularly, computing system 12 may train the machine learning model via a plurality of training features labeled with episodes of tremor, instead of the plurality of first (e.g. EEG waveforms) and second data labeled with episodes of tremor as described above.

Furthermore, computing system 12 can process the first and second data, particularly EEG data, to derive a classification of the episode of tremor. Further, computing system 12 can determine, for each of tremor type classification, class activation data indicating varying likelihoods of the classification over the period of time. For a given tremor type, an amplitude of such likelihood values at different times corresponds to a probability that a tremor is occurring at that time, with higher values corresponding to higher probability.

Particularly, computing system 12 can use class activation mapping to identify regions of an input time series, e.g., of EEG data, that constitute the reason for the time series being given a particular classification by the machine learning model of computing system 12. A class activation map for a given classification may be a univariate time series where each element (e.g., at each timestamp at the sampling frequency of the input time series) may be a weighted sum or other value derived from the outputs of an intermediate layer of a neural network or other machine learning model. The intermediate layer may be a global average pooling layer and/or last layer prior to the output layer neurons for each classification.

Furthermore, particularly, the computing device 12 may apply the machine learning model to other types of data to determine that an episode of tremor has occurred in patient P. For example, the computing system 12 can apply the machine learning model to one or more characteristics of EEG data that are correlated to tremor in the patient, an activity level of the implantable medical device 11, an input impedance of the implantable medical device 11, or a battery level of the implantable medical device 11.

Furthermore, processing circuitry 13 of computing system 12 may generate, from the first and second data, particularly from the EEG data, an intermediate representation of said data. For example, processing circuitry 13 may apply one or more signal processing, signal decomposition, wavelet decomposition, filtering, or noise reduction operations to said data (e.g. EEG data) to generate the intermediate representation of the respective data.

Particularly, computing system 12 processes such an intermediate representation of the data (e.g. EEG data) to detect and classify an episode of tremor in patient P. Furthermore, computing system 12 can train the machine learning model via a plurality of training intermediate representations labeled with episodes of tremor, instead of the plurality of raw first and second data (e.g. raw EEG waveforms) labeled with episodes of tremor as described above. The use of such intermediate representations of the first and second data may allow for the training and development of a less computationally involved machine learning model by computing system 12. Further, the use of such intermediate representations of the first and second data may require less iterations and fewer training data to build an accurate machine learning model, as opposed to the use of raw data to train the machine learning model.

Particularly, computing system 12 is configured to appy the machine learning model to the received first and second data (particularly EEG data) to detect an episode of tremor in patient P. In some examples, the machine learning model is trained with a plurality of EEG episodes annotated by a clinician or a monitoring center for tremor of several different types. Particularly, computing system 12 can be configured to apply the machine learning model to one or several subsegments of a normalized input EEG signal and to generate tremor labels and a likelihood of an occurrence of the tremor. Particularly, computing system 12 can be configured to determine, based on the machine learning model, that an episode of tremor has occurred in patient P and to determine a level of confidence in the determination that the episode of tremor has occurred in patient P. Particularly, computing system 12 can determine whether an episode of tremor of a plurality of different tremor types has occurred in patient P, as well as a level of confidence that an episode of tremor of each tremor type has occurred.

Furthermore, computing system 12 determines whether application of the machine learning model has detected an episode of tremor. In response to determining that an episode of tremor has been detected, computing system 12 determines whether the level of confidence in the determination is greater than a predetermined threshold. Particularly, the predetermined threshold is, e.g., 25%, 50%, 75%, 90%, 95%, 99%, etc. Particularly, computing system 12 determines whether the level of confidence in the determination is greater than a first predetermined threshold (e.g., 50%) and whether the level of confidence in the determination is greater than a second predetermined threshold (e.g., 90%). The first predetermined threshold may be associated with a medium level of confidence by computing system 12 that the episode of tremor has occurred, while the second predetermined threshold may be associated with a high level of confidence by computing system 12.

In response to determining that the level of confidence is greater than the predetermined threshold, computing system 12 can output the first and second data (e.g. EEG data) for review by a clinician. In some examples, computing system 12 can output a portion of the first data (e.g. EEG data), a first indication that the episode of tremor has occurred in patient P, and a second indication of the level of certainty in the determination that the episode of tremor has occurred in patient P. Furthermore, computing system 12 can selects a visualization method according to the level of confidence based on the machine learning module that the episode of tremor has occurred. For example, computing system 12 can apply color coding to indicate results for low, medium or high confidence that an episode of tremor has occurred.

Furthermore, computing system 12 particularly uses different visualization techniques to indicate a type of tremor. Particularly, computing system 12 can be configured to present first and second data and annotations thereto, particularly an EEG waveform and an annotation to the waveform, to indicate where the episode of tremor has occurred. In some examples, the annotation includes highlighting a section of the respective data, e.g. EEG, indicating a start and/or stop time of the episode of tremor, or applying a graphical icon or text to the section of the EEG. Computing system 12 can use a wide variety of different visualization techniques, such as color-coding, hatching, images or icons, shapes, indicators of different size, light, sound, textual notifications, etc. to provide information to the user.

Particularly, computing system 12 can display an indication that the episode of tremor that has occurred in the patient P and one or more of the features that coincide with the episode of tremor. Furthermore, computing system 12 can displays a classification of the episode of tremor as a particular type of tremor. Furthermore, particularly, computing system 12 can display a subsection of the EEG data or of other first and second data obtained from patient P that coincides with the episode of tremor. For example, computing system 12 can identify a subsection of the EEG data or of other first and second data of patient P, wherein the subsection comprises e.g. EEG data for a first time period prior to the episode of tremor, a second time period during the occurrence of the episode of tremor, and a third time period after the episode of tremor.

In response to determining that computing system 12 has not detected an episode of tremor based on the machine learning model, or in response to determining that the level of confidence is not greater than the predetermined threshold, computing system 12 is configured to store (e.g. in storage 10) the sensed first and second data (e.g. EEG data) for review by a monitoring center or clinician at a later time.

In response to determining that the computing system 12 has detected at least one episode of tremor of the selected type, computing system 12 is configured to determine a level of confidence based on the machine learning model that the episode of tremor of the selected type has occurred in patient P. Furthermore, particularly, computing system 12 is configured to output a portion of the first data (e.g. EEG data), a first indication that the episode of tremor has occurred in patient P, and a second indication of the level of certainty in the determination that the episode of tremor has occurred in patient P.

Furthermore, particularly, computing system 12 is configured to receive, from a user, a classification of an episode of tremor as being of a particular type of tremor. Particularly, computing system 12 is configured to use the received classification to train or update the machine learning model or the tremor threshold to increase the accuracy and performance of the machine learning model. This allows for increasing the accuracy and performance of the machine learning model / computing system 12 in detecting and classifying episodes of tremor of types that are difficult to detect, episodes of tremors of types that are dependent on a unique medical diagnosis particular to a particular patient, or episodes of tremor of types for which data is scarce, uncommon, or of low prevalence, or of tremor whose detection performance in a specific patient might be sub-optimal due to factors such as device position, change in physiological state, etc.

Furthermore, particularly, computing system 12 can be configured to determine whether a user type is advanced. Particularly, computing system 12 may customize the visualization of the EEG data (and particularly of other first and second data) based on the ability of the user. For example, computing system 12 can present more- or less-detailed EEG waveform, metadata, and resulting analysis provided by the machine learning model as needed for a variety of users of different abilities.

For example, in response to determining that the user type is not advanced (e.g., a basic user type), computing system 12 can be configured to output basic EEG data (or other first and second data), a first indication that the episode of tremor has occurred in patient P, and a second indication of the level of certainty in the determination that the episode of tremor has occurred in patient P. For example, for non-experts such as implanting cardiologists, neurologists, HF physicians, device nurses, patients/caregivers, computing system 12 can be configured to present for display an overall performance of the machine learning model in terms of simple indication of a positive detection or the absence of an episode of tremor. In some examples, computing system 12 can be further configured to present, for display, an EEG segment depicting the detected episode of tremor (and particularly also a segment of other first and second data) overlaid with a representative episode of tremor of the same classification. Furthermore, particularly, computing device 12 can be configured to present, to a basic user, an EEG waveform of the patient, a first representation of a first EEG waveform presenting an episode of tremor, and a second representation of a second EEG waveform presenting normal EEG / brain behavior.

Furthermore, particularly, in response to determining that the user type is advanced, computing system 12 can be configured to output advanced EEG data, the first indication that the episode of tremor has occurred in patient P, and the second indication of the level of certainty in the determination that the episode of tremor has occurred in patient P. Particularly, for an electrophysiologist or subject matter expert, computing system 12 can be configured to display, a start and stop time of each episode of tremor. Furthermore, particularly, computing system 12 may present additional types of information not expressly described herein to improve a confidence of the expert in the applied machine learning model and to assist the expert in interpreting an episode of tremor. Particularly, computing device 12 can be configured to present, to an advanced user, one or more of an EEG waveform of patient P, a start time of the episode of tremor, a stop time of the episode of tremor of patient P. Thus, the invention allows for a different data presentation based upon the skill, ability, or experience of the user accessing the data.

## Claims

1. A computing device (1), comprising:
a storage medium (10); and
processing circuitry (13) operably coupled to the storage medium (10) and configured to:
receive first data sensed by a medical device, and second data sensed by at least one wearable device
apply a machine learning model, trained using first and second data for a plurality of patients, to the received first and second data to:
determine, based on the machine learning model, that an episode of tremor has occurred in the patient; and
determine a level of confidence in the determination that the episode of tremor has occurred in the patient;
determine that the level of confidence in the determination that the episode of tremor has occurred in the patient is greater than a predetermined threshold; and in response to determining that the level of confidence is greater than the predetermined threshold, output, for display to a user, at least a portion of the first data, a first indication that the episode of tremor has occurred in the patient, and a second indication of the level of confidence that the episode of tremor has occurred in the patient.

2. The computing system according to claim 1, wherein the first data comprises at least one of: data indicative of neural activity, an electroencephalogram (EEG) waveform, an electrocortigram (ECoG) waveform.

3. The computing system according to claim 1 or 2, wherein the second data comprises one of: data concerning motion, temperature, blood pressure.

4. The computing system according to one of the preceding claims, wherein the at least one portion of the first data comprises an electroencephalogram (EEG) waveform, and wherein the first indication that the episode of tremor has occurred in the patient comprises an annotation to the EEG waveform.

5. The computing system according to one of the preceding claims, wherein the second indication comprises one or more of: a color, an image, a light, a sound, a textual notification.

6. The computing system according to one of the preceding claims, wherein the processing circuitry (13) is further configured to receive, from the user, a selection of a tremor type, wherein to apply the machine learning model to the received first and second data to determine that an episode of tremor has occurred in the patient, the processing circuitry is configured to apply the machine learning model to the received first and second data to determine that an episode of tremor of the selected tremor type has occurred in the patient, and wherein to output the at least one portion of the first data, the first indicator that the episode of tremor has occurred in the patient, and the second indicator of the level of confidence that the episode of tremor has occurred in the patient, the processing circuitry is configured to output the at least one portion of the first data, a first indicator that the episode of tremor of the selected tremor type has occurred in the patient, and the second indicator of the level of confidence that the episode of tremor of the selected tremor type has occurred in the patient.

7. The computing system according to one of the claims 1 to 6, wherein the processing circuitry (13) is configured to determine that the user is a basic user, wherein, to output the at least one portion of the first data, the first indicator that the episode of tremor has occurred in the patient, and the second indicator of the level of confidence that the episode of tremor has occurred in the patient, the processing circuitry is configured to output, in response to determining that the user is a basic user, the first indicator that the episode of tremor has occurred in the patient, the second indicator of the level of confidence that the episode of tremor has occurred in the patient, and one or more of: an electroencephalogram (EEG) waveform of the patient; a first representation of a first EEG waveform presenting an episode of tremor; and a second representation of a second EEG waveform presenting normal behavior.

8. The computing system according to one of the claims 1 to 7, wherein the processing circuitry (13) is configured to determine that the user is an advanced user, wherein, to output the at least one portion of the first data, the first indicator that the episode of tremor has occurred in the patient, and the second indicator of the level of confidence that the episode of tremor has occurred in the patient, the processing circuitry (13) is configured to output, in response to determining that the user is an advanced user, the first indicator that the episode of tremor has occurred in the patient, the second indicator of the level of confidence that the episode of tremor has occurred in the patient, and one or more of: an electroencephalogram (EEG) waveform of the patient; a start time of the episode of tremor; a stop time of the episode of tremor.

9. The computing system according to one of the claims 1 to 8, wherein the machine learning model trained using first and second data for the plurality of patients comprises a machine learning model trained using a plurality of electroencephalogram (EEG) waveforms, each EEG waveform labeled with one or more episodes of tremor in a patient of the plurality of patients.

10. The computing system according to one of the claims 1 to 9, wherein to apply the machine learning model to the received first and second data, the processing circuitry (13) is configured to apply the machine learning model to at least one of:
electroencephalogram (EEG) data of the patient; the characteristics correlated to tremor in the patient; a type of the tremor in the patient; an activity level of the implantable medical device; an input impedance of the implantable medical device (11); a battery level of the implantable medical device (11); motion data correlated to tremor in the patient; temperature of the patient correlated to tremor in the patient, blood pressure correlated to tremor in the patient.

11. The computing system according to one of the claims 1 to 10, wherein to output the at least one portion of the first data, the processing circuitry is configured to:
identify a subsection of an electroencephalogram (EEG) of the patient, wherein the subsection comprises EEG data for a first time period prior to the episode of tremor, a second time period during the episode of tremor, and a third time period after the episode of tremor, and wherein a length of time of the EEG of the patient is greater than the first, second, and third time periods; and output the subsection of the EEG.

12. The computing system according to one of the claims 1 to 11, wherein the predetermined threshold is a first predetermined threshold, and wherein the processing circuitry is further configured to determine whether the level of confidence in the determination that the episode of tremor has occurred in the patient is greater than a second predetermined threshold, the second predetermined threshold greater than the first predetermined threshold, wherein in response to determining that the level of confidence is greater than the predetermined threshold, to output, the at least one portion of the first data, the first indication, and the second indication, the processing circuitry is configured to: in response to determining that the level of confidence is greater than the first predetermined threshold but not greater than the second predetermined threshold, output, the at least one portion of the first data, the first indication, and an indication of a medium level of confidence that the episode of tremor has occurred in the patient; in response to determining that the level of confidence is greater than the first predetermined threshold and greater than the second predetermined threshold, output, the at least a portion of the first data, the first indication, and an indication of a high level of confidence that the episode of tremor has occurred in the patient.

13. A system, comprising:
means for receiving, by a computing system (12) comprising processing circuitry (13) and a storage medium (10), first data sensed by a medical device (11) and second data sensed by a wearable device (15);
means for applying, by the computing system (12), a machine learning model, trained using first and second data for a plurality of patients, to the received first and second data to:
determine, based on the machine learning model, that an episode of tremor has occurred in the patient (P); and
determine a level of confidence in the determination that the episode of tremor has occurred in the patient (P);
means for determining that the level of confidence in the determination that the episode of tremor has occurred in the patient is greater than a predetermined threshold; and
means for outputting, by the computing system (12) and for display to a user, at least a portion of the first data, a first indication that the episode of tremor has occurred in the patient (P), and a second indication of the level of confidence that the episode of tremor has occurred in the patient (P).

14. A method, comprising:
Receiving, by a computing system (12) comprising a storage medium (10) and
processing circuitry (13) operably coupled to the storage medium (10),
first data sensed by a medical device (11), and second data sensed by at least one wearable device (15);
applying, by the computing system (12), a machine learning model, trained using first and second data for a plurality of patients, to the received first and second data to:
determine, based on the machine learning model, that an episode of tremor has occurred in the patient; and
determine a level of confidence in the determination that the episode of tremor has occurred in the patient;
determining that the level of confidence in the determination that the episode of tremor has occurred in the patient is greater than a predetermined threshold; and in response to determining that the level of confidence is greater than the predetermined threshold, outputting, by the computing system and for display to a user, at least a portion of the first data, a first indication that the episode of tremor has occurred in the patient, and
a second indication of the level of confidence that the episode of tremor has occurred in the patient.

15. The method according to claim 14, wherein the first data comprises at least one of: data indicative of neural activity, an electroencephalogram (EEG) waveform, an electrocortigram (ECoG) waveform.
